(19) Europäisches Patentamt
European Patent Office
Office européen des brevets

(11) **EP 1 713 431 B1**

(12) **EUROPEAN PATENT SPECIFICATION**

(45) Date of publication and mention
of the grant of the patent:
**15.04.2009 Bulletin 2009/16**

(51) Int Cl.:
**A61J 1/10** (2006.01)

(21) Application number: **05704817.5**

(86) International application number:
**PCT/SE2005/000173**

(22) Date of filing: **10.02.2005**

(87) International publication number:
**WO 2005/074861 (18.08.2005 Gazette 2005/33)**

(54) **A FLUID BAG, USE OF ONE OR MORE FLUID BAGS AND A SYSTEM INCLUDING ONE OR MORE FLUID BAGS**

FLUIDBEUTEL, VERWENDUNG VON EINEM ODER MEHREREN FLUIDBEUTELN UND SYSTEM MIT EINEM ODER MEHREREN FLUIDBEUTELN

POCHE A LIQUIDE, UTILISATION D'UNE OU PLUSIEURS POCHES A LIQUIDE ET SYSTEME COMPORTANT UNE OU PLUSIEURS POCHES A LIQUIDE

(84) Designated Contracting States:
**AT BE BG CH CY CZ DE DK EE ES FI FR GB GR HU IE IS IT LI LT LU MC NL PL PT RO SE SI SK TR**

(30) Priority: **10.02.2004 SE 0400271**
**13.02.2004 US 543920 P**

(43) Date of publication of application:
**25.10.2006 Bulletin 2006/43**

(73) Proprietor: **Gambro Lundia AB**
**220 10 Lund (SE)**

(72) Inventors:
• **HAGERMARK, Michaela**
**S-234 32 Lomma (SE)**
• **PAOLINI, Francesco**
**I-41010 Modena (IT)**

(74) Representative: **Bornegard, Annette et al**
**Gambro Lundia AB**
**Patent Department,**
**P.O. Box 10101**
**220 10 Lund (SE)**

(56) References cited:
**EP-A1- 0 711 536**          **EP-A2- 1 002 512**
**WO-A1-91/00074**          **GB-A- 2 080 116**
**US-A- 4 096 897**          **US-A- 4 100 953**
**US-A- 5 364 385**          **US-A- 5 632 738**

## Description

BACKGROUND OF THE INVENTION

The invention relates to renal care. More precisely the present invention concerns a fluid bag that can be used in connection with an apparatus for hemodialysis, hemodiafiltration, hemofiltration or peritoneal dialysis. The invention also concerns the use of one or more such fluid bags and to a system including one or more such fluid bags.

Hemodialysis is a treatment designed to correct the chemical composition of blood by removing accumulated metabolic products and adding buffer in a process of diffusion and/or convection through a natural or synthetic semi-permeable membrane.

A conventional kind of hemodialysis apparatus is well known to a person skilled in the art. An example of such an apparatus is described in connection with Fig 1 in EP-A2-904 789. A hemodialysis apparatus is used to treat a patient suffering from kidney failure. In a dialysis apparatus, a dialysis fluid (dialysis solution) is prepared. The dialysis fluid is used to achieve dialysis in a dialyser that is part of the hemodialysis apparatus. The dialysis fluid can be prepared in the apparatus by feeding water and one or more concentrates to the apparatus. It is also known to provide the dialysis fluid to the apparatus without feeding water to the apparatus. In this case the dialysis fluid that is fed to the apparatus does not have to be diluted in the apparatus. The dialysis fluid fed to the apparatus is thus already of a suitable concentration. The dialysis fluid may however still be fed from a plurality of containers, having fluids of different compositions, such that the fluids from the different containers are mixed in the apparatus to obtain the final dialysis fluid. The apparatus may also be arranged to provide the patient with a replacement fluid to replace the ultrafiltrate withdrawn from the patient. Such a replacement fluid may be the same as or different than the dialysis fluid. Since a hemodialysis apparatus is well known to a person skilled in the art, it will not be described in further detail here.

Hemofiltration is a treatment designed to remove accumulated metabolic products from blood by the process of convective transport as a consequence of ultrafiltration through a semi-permeable membrane of high-flux type; the volume of filtered fluid exceeding the desired weight loss is replaced by sterile pyrogen-free infusion solution. In a pure hemofiltration process, normally no dialysis fluid is used.

Hemodiafiltration is a treatment designed to remove accumulated metabolic products from blood by a combination of diffusive and convective transport through a semi-permeable membrane of high-flux type; fluid is removed by ultrafiltration and the volume of filtered fluid exceeding the desired weight loss is replaced by sterile, pyrogen-free replacement fluid.

There exist apparatuses that can be used for both hemodialysis and hemofiltration, as well as for hemodiafiltration.

There also exist apparatuses for peritoneal dialysis. In peritoneal dialysis no dialyser that is part of an apparatus is needed. Instead the peritoneum of the patient is used as a dialysis membrane. Also in an apparatus for peritoneal dialysis, a dialysis fluid and/or a replacement fluid is added.

The rest product (e.g. the dialysate or ultrafiltrate) from a process in which an apparatus of the above described kind is used can be fed from the apparatus to a sewage system. It is also known to collect the rest product in a container, for example in the form of a fluid bag, connected to the apparatus.

The dialysis fluid, or the concentrate that forms the basis for the dialysis fluid, or the replacement fluid, can for example be provided in a fluid bag. The fluid bag may contain one or more compartments. An example of a fluid bag containing a dialysis fluid is known from WO 99/27885 A1.

It may also be mentioned that fluid bags are known to be used in other medical fields than the field to which the present invention pertains. For example US 5,364,385 discloses a fluid bag for blood or blood components. The fluid bag contains about 300 ml. This bag is made rather thin for the purpose of efficiently thawing frozen blood components included in the bag. The bag comprises a number of seal lines for constraining the expansion of the bag.

EP 711 536 A1 describes a urinary drainage bag. The bag can be worn by a patient over the abdomen beneath the clothing. The bag includes a number of heat seals. These heat seals maintain the bag in relatively flat condition and impede the surge of its liquid contents from one side to the other as the patient moves about.

SUMMARY OF THE INVENTION

An object of the present invention is to provide a fluid bag that contains a dialysis, replacement or rest product fluid as defined below and that is advantageous to use in connection with an apparatus for hemodialysis, hemodiafiltration, hemofiltration or peritoneal dialysis. Advantageously, but not necessarily, the fluid bag should be suitable to be used in connection with such an apparatus which is not connected to a water supply pipe or to a sewage system. A further object is to provide such a fluid bag that can be arranged in an advantageous manner on or next to such an apparatus. In particular, it is an object that the fluid bag should be such that a plurality of fluid bags that are useful for this purpose can be arranged in a rather limited space. Still another object is that the fluid bag should be easy to handle.

The above objects are achieved by a fluid bag that comprises one or more compartments suitable for containing one or more fluids.

The fluid bag as claimed contains, in at least one compartment, or in a combination of different compartments, either a dialysis fluid, suitable for use as a dialysis fluid in an apparatus for hemodialysis, hemodiafiltration, hemofiltration or peritoneal dialysis, or a replacement fluid, suitable to be delivered to a patient in order to replace the ultrafiltrate that is withdrawn from a patient by a process of hemodialysis, hemofiltration, hemodiafiltration or peritoneal dialysis, or a fluid that is a rest product from a process of hemodialysis, hemofiltration, hemodiafiltration or peritoneal dialysis. The fluid bag is at least partly made of a flexible material and comprises a first main sheet and a second main sheet located opposite to said first main sheet, the distance between said first and second sheets defining the thickness of the fluid bag. The fluid bag has a size such that it contains at least a certain quantity q ml of fluid when it is completely filled with one or more fluids. The fluid bag is provided with one or more distance limiting members, arranged to limit the distance between said first and second sheets, such that the thickness of the fluid bag, when the fluid bag is suspended such that it hangs vertically, never exceeds a certain value v mm independently of whether the fluid bag is completely full, completely empty or filled to any degree there between. The fluid bag is such that q ≥ 2000, and v ≥ 2q/100.

Such a fluid bag has several advantages. The fluid bag is sufficiently large, such that it can contain an appropriate amount of fluid, for example of a dialysis fluid, even if this fluid is not in a concentrated form. It is therefore not necessary to exchange the fluid bag so often. Furthermore, the fluid bag is arranged such that it is not too thick. It is thereby possible to easily arrange a plurality of fluid bags next to each other in connection with an apparatus of the above mentioned kind. For this reason, a sufficient supply of such fluid bags may be held in position on an apparatus or close to an apparatus. It is thereby not necessary to supply the apparatus with new fluid bags so often. This is advantageous since it makes it possible for for example a nurse to administer the work involved with the supply of fluid bags in an efficient manner. Furthermore, because of the above defined relationship between volume and thickness, the fluid bags according to the invention are easy to handle.

It may be noted that the thickness of the fluid bag can be measured as the horizontal distance between corresponding points on said first and second sheets when the fluid bag hangs freely vertically from a support, i.e. without being constrained to a limited space. The distance is thereby measured between the corresponding points on said first and second sheets where the distance is the largest. The concept thickness is thus used as the concept is normally used. It may also be noted that this thickness of the fluid bag may depend on how full the fluid bag is with fluid. It is for example feasible that when the fluid bag is completely full the thickness is somewhat lower than when the fluid bag is filled to for example 60%. Therefore, the value v is defined such that the thickness never exceeds this value independently of how much fluid the fluid bag contains. The mentioned fluid that is a rest product may for example be a dialysate (used dialysis fluid), ultrafiltrate or unused dialysis fluid.

According to a preferred embodiment, v ≤ 0.0175 q, preferably v ≤ 0.016 q. According to these embodiments, the fluid bag is advantageously thin in relation to its volume. Therefore, several fluid bags may be positioned next to each other in a rather limited space. These fluid bags are also easier to handle.

Advantageously, q ≥ 3000 or q ≥ 4000. Since, according to these embodiments, the fluid bag can contain a relatively large amount of fluid, the fluid bag does not have to be changed very often. The fluid bags may also be able to contain a larger quantity than what has previously been defined. For example, each fluid bag can have a volume such that it can contain between 4500 ml and 5500 ml. A fluid bag according to the invention may even contain larger quantities than this. However, in case the fluid bag contains too much fluid, the fluid bag may be more difficult to handle. Furthermore, the strain on the fluid bag will become larger, the more fluid it contains.

The fluid bag is provided with one or more distance limiting members, arranged to limit the distance between said first and second sheets. By providing such distance limiting members, it is possible to control in an efficient manner that the maximum thickness never exceeds the value v.

According to an advantageous embodiment, the extension of said first sheet is limited by a first boundary of said first sheet, and the extension of said second sheet is limited by a second boundary of said second sheet and wherein at least one such distance limiting member is formed by fastening said first sheet to said second sheet at a position located at least at a distance from said first and second boundaries, and, if the bag includes a plurality of compartments, at a distance from the borderlines between the different compartments. By arranging the distance limiting members in such positions, the maximum thickness can be controlled in a rather efficient and simple manner.

A distance limiting member can according to a preferred embodiment be formed by a weld joining said first and second sheets. The weld can have the shape of a substantially straight line or can have the shape of a loop, for example a circular or oval loop. By forming the distance limiting members as welds, these distance limiting members can be produced in a relatively simple and inexpensive manner. Welds in the form of straight lines or in the form of loops are easy to produce and work to limit the maximum thickness in an efficient way. It should however be noted that the distance limiting members can be formed in other manners and they do also not have to have the shape of a straight line or a loop. For example, the distance limiting members may also be formed by welds that form a curved line.

Preferably, the fluid bag includes a plurality of said distance limiting members, for example at least three, or at least four, distance limiting members. By using several distance limiting members, the maximum thickness can be controlled more efficiently.

According to an advantageous embodiment, the fluid bag includes at least two compartments. Each of at least two compartments can be provided with at least one distance limiting member. When the fluid bag includes a plurality of compartments, for example different components of a dialysis fluid can be stored in different compartments in a single fluid bag. This is advantageous if the different components cannot be stored for a longer time in a mixed state. The contents of the different compartments can then be mixed before the fluid in the bag is used. This is explained for example in the above cited WO 99/27885 A1. By providing distance limiting members in different compartments, it can be ensured that the value v is never exceeded even if the compartments are relatively large. According to one possible embodiment, the fluid bag comprises at least three compartments. Thereby at least three different fluids can be arranged in different compartments in the fluid bag. The fluid bag can, for example, have exactly three compartments. However, it is also possible that the fluid bag has more than three compartments. The above mentioned distance limiting members can thereby be arranged in one of the compartments, in a plurality of compartments or in each of the compartments. If the fluid bag includes several compartments, and if the borderlines between the different compartments are located appropriately, it may not be necessary to arrange distance limiting members in each compartment in order to obtain a sufficiently thin fluid bag in accordance with the invention. In particular, if a compartment is rather small it may not be necessary to arrange any distance limiting member in such a compartment. If the number of compartments are sufficiently large and/or if the borderlines between the different compartments are appropriately arranged, then it may even not be necessary to arrange a distance limiting member in any compartment, since the borderlines themselves can ensure that the fluid bag is sufficiently thin in accordance with the invention.

According to a preferred embodiment, the fluid bag has a first edge portion, wherein the fluid bag is provided with attachment means, located at said first edge portion, for attaching the fluid bag to holding means, suitable to hold the fluid bag in a suspended position. The attachment means can for example be formed by at least one hole through said first edge portion. By providing the fluid bag with one or more holes, it is easy to attach the fluid bag to the holding means. The holding means may for example have holding members in the form of hooks such that the hooks can extend through the holes in said first edge portion.

The invention also relates to the use of one of more fluid bags according to any of the preceding embodiments. At least one such fluid bag can thereby be connected, via a conduit, to an apparatus for hemodialysis, hemodiafiltration, hemofiltration or peritoneal dialysis. In accordance with what has been said above, it is advantageous to use a fluid bag according to the invention in this manner.

Preferably, one or more such fluid bags are suspended by holding means such that the fluid bag hangs down from said holding means. The fluid bag can for example be of the above described kind that is provided with at least one hole in an edge portion, wherein said holding means holds the fluid bag by a holding member protruding through said hole. The holding member may thus for example be formed as a hook that can extend through the hole in the fluid bag.

The holding means preferably forms part of or is arranged in proximity to an apparatus for hemodialysis, hemodiafiltration, hemofiltration or peritoneal dialysis. Thereby, a plurality of fluid bags may be provided at, or in the immediate vicinity of, an apparatus of this kind. It may be noted that the expression "in proximity to an apparatus" here preferably means that the fluid bags are so close to the apparatus that the fluid bags can be connected to the apparatus by a normal conduit that is used for connecting fluid bags of this kind to an apparatus of the above described kind.

A plurality of fluid bags, for example at least two, at least three or at least four fluid bags, are preferably arranged suspended from said holding means such that the fluid bags are arranged after each other as seen in the thickness direction of the fluid bags. Each fluid bag can thereby be attached to said holding means at a certain position at said holding means, such that the distance d mm between the positions for neighbouring fluid bags is such that $d \geq v$. For example, $v < d < 1.5\ v$ or $v < d < 1.2\ v$. By arranging a plurality of fluid bags in this manner, the fluid bags do not require too much space and can be positioned close to each other. It should be noted that although the mentioned distance preferably is larger or equal than v, it is also within the scope of the invention that the mentioned distance d may even be somewhat less than v. This means that the fluid bags, when they have their maximum thickness, will touch each other when they are arranged next to each other. However, by making the distance d at least equal to v, or preferably larger than v, there is enough space such that the fluid bags may be arranged suspended without touching each other.

Another aspect of the invention relates to a system for hemodialysis, hemodiafiltration, hemofiltration or peritoneal dialysis. The system hereby comprises an apparatus for hemodialysis, hemodiafiltration, hemofiltration or peritoneal dialysis and at least one fluid bag according to any of the above embodiments. With such a system, the advantages described above will be achieved.

The system preferably comprises holding means that forms part of or is arranged in proximity to said apparatus for hemodialysis, hemodiafiltration, hemofiltration or peritoneal dialysis, wherein one or more fluid bags are suspended by said holding means, such that the fluid bag hangs down from said holding means.

The fluid bags can preferably be arranged in the system in the same manner as explained above in connection with the use of one or more fluid bags.

Further examples of the present invention will become clear from the description below and from the appended claims.

BRIEF DESCRIPTION OF THE DRAWINGS

Fig.1 shows a schematic front view of a typical fluid bag according to the prior art.

Fig. 2 shows a schematic side view in the direction II-II of the fluid bag of Fig. 1.

Fig. 3 shows a schematic front view, similar to the view of Fig. 1, of a fluid bag, generally according to the prior art, comprising two compartments.

Fig. 4 shows a schematic front view of an embodiment of a fluid bag according to the present invention.

Fig. 5 shows a schematic side view of the fluid bag of Fig. 4 in the direction V-V.

Fig. 6 shows a schematic front view of another embodiment of a fluid bag according to the present invention.

Fig. 7 shows a schematic front view of still another embodiment of a fluid bag according to the invention.

Fig. 8 shows a schematic front view of a further embodiment of a fluid bag according to the present invention.

Fig. 9 shows schematically a front view of a system according to the present invention including a dialysis apparatus and a number of fluid bags.

DESCRIPTION OF PREFERRED EMBODIMENTS

It should be noted that when in this document the concept dialysis or the concept dialysis apparatus is mentioned, these concepts are meant to refer to hemodialysis, hemodiafiltration, hemofiltration or peritoneal dialysis. It should also be noted that the same reference signs are used for the corresponding parts in the different figures.

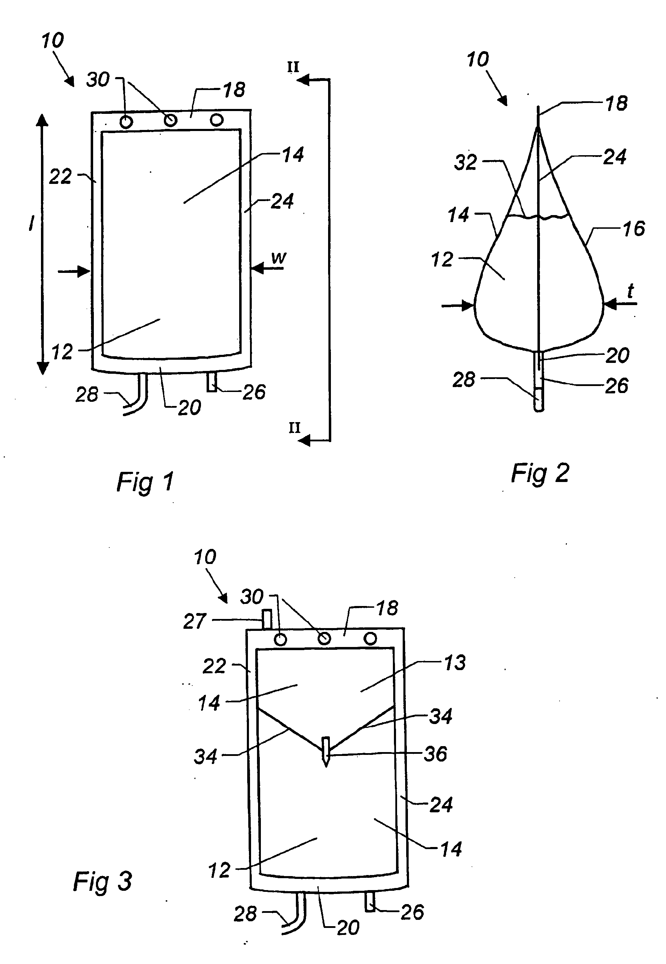

With reference to Fig. 1 and 2 a fluid bag according to the prior art will be described. The fluid bag 10 is a fluid bag 10 for a dialysis fluid or for a replacement fluid. A similar fluid bag 10 may also be used to collect a waste fluid that is a rest product from a process of dialysis. The rest product may for example be a dialysate (used dialysis fluid), ultrafiltrate or unused dialysis fluid.

The fluid bag 10 has one compartment 12 for a fluid. The fluid bag 10 can be formed of a suitable flexible material, for example PVC or non-PVC such as polyolefin and thermoplastic elastomer. The fluid bag 10 is formed by a first main sheet 14 made of a flexible material and a second main sheet 16 also made of a flexible material. The fluid bag 10 can for example be formed by welding these sheets 14, 16 together along a first 18, a second 20, a third 22 and a fourth 24 edge portion. The extension of the first sheet 14 is thus limited by a first boundary which is formed by said first 18, second 20, third 22 and fourth 24 edge portions. It should however be noted that the fluid bag 10 can also be formed of a tubular material, which for example is welded to form said first 18 and second 20 edge portions. In such a fluid bag 10, formed by a tubular material, there is thus no distinct third 22 and fourth 24 edge portions that include a weld. In this case, the extension of the first sheet 14 can be seen to be limited by a first edge portion 18, a second edge portion 20 and by further imaginary boundaries between the first sheet 14 and the second sheet 16, which imaginary boundaries thus are located at positions corresponding to the third 22 and fourth 24 edge portions in Fig. 1 and 2. According to still another possible design of the fluid bag 10, said first 14 and second 16 sheets do not have to be connected directly to each other. Instead there could be additional sides which join the first 14 and second 16 sheets.

The fluid bag 10 is provided with an inlet tube 26, via which a fluid can be introduced into the fluid bag 10. The fluid bag 10 also has an exit conduit 28, via which a fluid can be fed from the fluid bag 10 to a dialysis apparatus. It should be mentioned that it is of course also possible to use one and the same member (for example the conduit 28) both for introducing a fluid into the fluid bag 10 and for feeding a fluid from the fluid bag 10. It is thus possible that the fluid bag 10 is provided with only one such member 28.

According to the shown embodiment, the first edge portion 18 has a number of holes 30 for attaching the fluid bag 10 to some holding means, for example in the form of hooks, such that the fluid bag 10 can be suspended from such holding means.

The fluid bag 10 has a length I and a width w. The length I can for example be about 500 mm and the width w can be about 350 mm. As seen in Fig. 2, the fluid bag 10 has a thickness t. How thick the fluid bag 10 is, may depend on how much fluid the fluid bag 10 contains. Since the first sheet 14 and the second sheet 16 are made of a flexible material, the fluid bag 10 can be rather thick when it is partly or completely filled with a fluid. The thickness t of the fluid bag 10 may make the fluid bag 10 difficult to handle. Fig. 2 indicates that the fluid bag 10 is filled with a fluid up to the level 32. The shown thickness t for a fluid bag according to the prior art can be about 130 mm.

Fig. 3 shows a view similar to the view of Fig. 1 of another example of a fluid bag 10 generally according to the prior art. However, this fluid bag 10 comprises two different compartments 12, 13. This fluid bag 10 has two inlet tubes 26, 27 such that a fluid can be introduced into both compartments 12, 13. The different compartments 12, 13 may contain different fluids that are to be mixed before the final dialysis fluid is formed. The different fluids may for example have such components that it is difficult to store the fluids in a mixed state for a longer time. Borderlines 34 indicate the borders between the compartments 12, 13. Before the dialysis fluid is fed to an apparatus or to a patient, the contents of the two compartments 12, 13 are to be mixed. This can for example be done in that the fluid bag 10 includes a frangible pin 36 that is normally closed, but that can be opened when the contents in the two compartments 12, 13 are to be mixed. The fluid bag 10 can of course be provided with any other means, instead of a frangible pin 36, for mixing the contents of the different compartments 12, 13. The fluid bag 10 may for example be provided with a so-called peelseal.

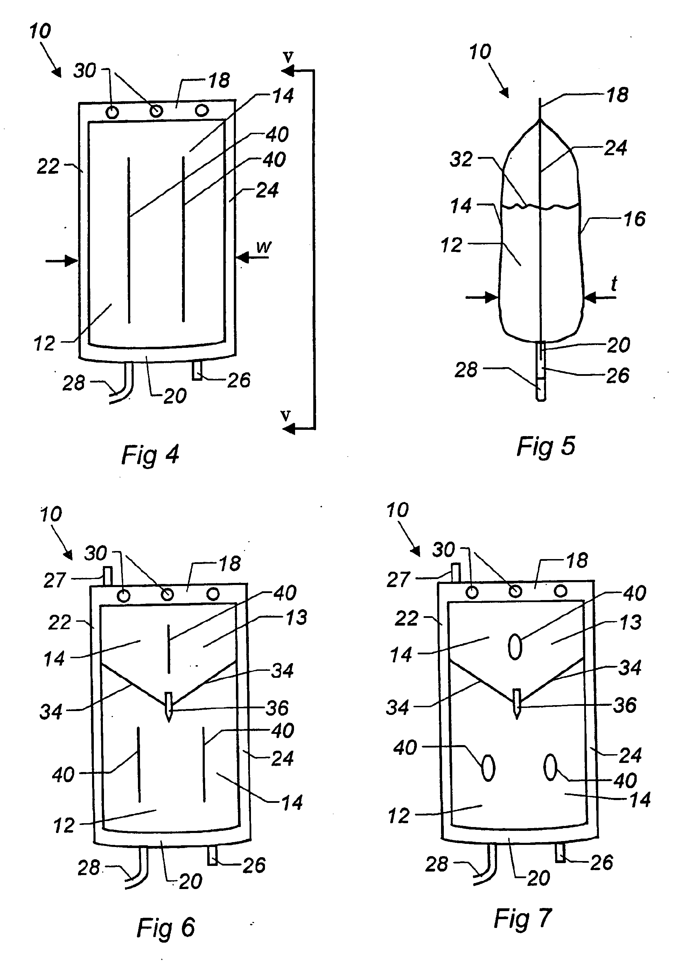

With reference to Fig. 4 and 5, a first embodiment of a fluid bag according to the present invention will now be described. A fluid bag according to the invention may for example be used in connection with high flow treatment. According to this embodiment, the fluid bag 10 comprises only one compartment 12 and is similar to the fluid bag 10 shown in Fig. 1 and 2. The fluid bag 10 contains a dialysis fluid suitable for use as a dialysis fluid in an apparatus for hemodialysis, hemodiafiltration, hemofiltration or peritoneal dialysis, or a replacement fluid, suitable to be delivered to a patient in order to replace the ultrafiltrate that has been drawn from a patient by a process of hemodialysis, hemofiltration, hemodiafiltration or peritoneal dialysis, or a fluid that is a rest product from a process of hemodialysis, hemofiltration, hemodiafiltration or peritoneal dialysis. Similar to the fluid bag 10 described in connection with Fig. 1 and 2, also the fluid bag 10 according to the invention comprises a first main sheet 14 made of a flexible material and a second main sheet 16 also made of a flexible material. In the same manner as described above, the distance between the first 14 and second 16 sheets defines the thickness t of the fluid bag 10. The fluid bag 10 has a size such that it contains a quantity q ml of fluid when it is completely filled with fluid. The quantity $q \geq 2000$, preferably $\geq 3000$ or $\geq 4000$. A most preferred size of the fluid bag 10 is that it can contain a quantity of fluid that is between 4500 ml and 5500 ml.

The fluid bag 10 according to the invention is designed such that its thickness never exceeds a certain thickness, v mm, independently of to what degree the fluid bag 10 is filled with the fluid. According to the invention, $v \leq 2 \, q/100$, preferably $v \leq 0.0175 \, q$, most preferred $v \leq 0.016 \, q$. This can be obtained in different manners. According to a preferred embodiment, the fluid bag 10 comprises one or more distance limiting members 40. Fig. 4 indicates two such distance limiting members 40. The distance limiting members 40 can for example be formed by welding the first sheet 14 to the second sheet 16 at certain positions. Fig. 4 discloses two such welds 40 in the form of straight lines. The distance limiting members 40 thus prevent the fluid bag 10 from becoming too thick. Fig. 5 shows that the fluid bag according to the invention has a thickness t that is essentially reduced compared to the thickness t of a fluid bag according to the prior art as shown in Fig. 2.

Fig. 6 shows a similar schematic front view as Fig. 4 of another embodiment of the invention. The fluid bag 10 according to this embodiment comprises two compartments 12, 13 and is thus similar to the fluid bag 10 shown in Fig. 3. In this case, the fluid bag 10 is provided with three distance limiting members 40 in the form of straight lines. The distance limiting members 40 may even in this case be formed by welding the first sheet 14 and the second sheet 16 together along the indicated straight lines 40.

Fig. 7 shows a front view of still another embodiment of the present invention. The fluid bag 10 according to this embodiment also includes two compartments 12, 13. In this case, the distance limiting members 40 have the shape of loops. These distance limiting members 40 can also in this case be formed by welding the first sheet 14 and the second sheet 16 together along the indicated loops 40.

It may be noted that in the embodiments of Fig. 4-7, the distance limiting members 40 are arranged such that they are located at a distance from the boundaries of the first 14 and second 16 sheets and also at a distance from the borderlines 34 between the different compartments 12, 13.

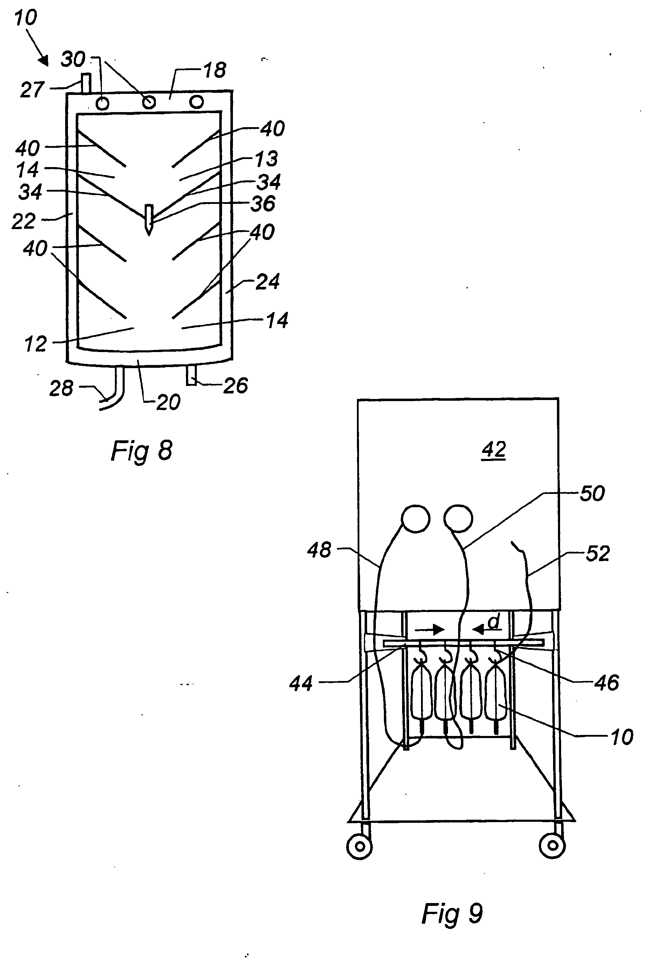

Fig. 8 shows a front view of another embodiment of the invention. Also according to this embodiment, the fluid bag 10 comprises two compartments 12, 13. In this case, the distance limiting members 40 are formed by welds. However, according to this embodiment, the welds 40 reach all the way to the boundaries of the first 14 and second 16 sheets.

It should be noted that a fluid bag 10 according to the present invention may also comprise more than two compartments 12, 13. Furthermore, the distance limiting members 40 do not necessarily have to be formed by welds, through which the first 14 and second 16 sheets are welded together. According to an alternative embodiment, the distance limiting members 40 could be formed by some members of a certain length. Each member could then have two ends: a first end attached to the first sheet 14 and the second end attached to the second sheet 16. It may also be noted that in case the distance limiting members 40 are formed by welds, the welds can have different configurations than those shown in the figures. The number of distance limiting members 40 may also vary. If the fluid bag 10 comprises at least two compartments 12, 13, then, as indicated in Fig. 6, 7 and 8, distance limiting members 40 may be arranged in the different compartments 12, 13. However, it is also within the scope of the invention that distance limiting members 40 are arranged only in one of the compartments 12, 13, for example in the largest compartment 12.

Similar to the fluid bag 10 shown in Fig. 1, the fluid bag 10 according to the invention preferably includes attachment means 30 for attaching the fluid bag 10 to holding means, suitable to hold the fluid bag in a suspended position. These attachment means 30 can be formed for example by one or more holes 30. The holes 30 may for example be arranged in the mentioned first edge portion 18.

Fig. 9 schematically shows a front view of a system according to the invention. At the same time, Fig. 9 indicates a manner of using one or more fluid bags 10 according to the invention. Fig. 9 thus shows an apparatus 42. This apparatus 42 can be an apparatus 42 for hemodialysis, hemodiafiltration, hemofiltration or peritoneal dialysis. The system also includes a number of fluid bags 10. The fluid bags 10 contain a dialysis fluid, a replacement fluid or a fluid that is a rest product as explained above. The apparatus 42 comprises holding means 44. The holding means 44 in this case forms part of the apparatus 42. However, the holding means 44 could instead be arranged next to the apparatus 42. In the shown embodiment, the holding means 44 are arranged below the actual apparatus 42. However, the holding means 44 could instead be arranged above the apparatus 42 or to the side of the apparatus 42. The fluid bags 10 are suspended from the holding means 44 such that the fluid bags 10 hang down from the holding means 44. The holding means 44 has a plurality of holding members 46 arranged to hold the fluid bags 10. The holding members 46 may for example be in the form of hooks that can be introduced through the holes 30 at the first edge portion 18 of the fluid bags 10. The holding members 46 can have fixed positions on the holding means 44. Alternatively, the holding members 46 can be movable along the holding means 44. The distance between neighbouring holding members 46 is indicated with d. Preferably, $d \geq v$. For example, $v < d < 1.5$ v or preferably $v < d < 1.2$ v. In this manner, the fluid bags 10 have sufficient room to be freely suspended from the holding members 46. Since the fluid bags 10 according to the present invention are made to be relatively thin, but still contain a large quantity of fluid, a number of fluid bags 10 can be efficiently arranged to suspend from the holding means 44.

Fig. 9 indicates that a first fluid bag 10 is connected to the apparatus 42 via a conduit 48. This fluid bag 10 may for example contain a dialysis fluid. Fig. 9 also indicates that a second fluid bag 10 is connected to the apparatus 42 via a conduit 50. This fluid bag 10 may for example contain a replacement fluid. The replacement fluid may be the same as the dialysis fluid. A third fluid bag 10 is connected to the apparatus 42 via a conduit 52. This conduit 52 may for example be a conduit for waste fluid from the apparatus 42. This fluid bag 10 thus contains a fluid in the form of a rest product from the process performed by the apparatus 42. Fig. 9 shows another fluid bag 10 that is not connected to the apparatus 42. This fluid bag 10 is stored for future use. For example, when all the dialysis fluid in the fluid bag 10 connected via the conduit 48 has been used, the fourth fluid bag 10 (that in Fig. 9 is not connected via a conduit to the apparatus 42) may instead be connected via a conduit to the apparatus 42. The number of fluid bags 10 suspended from the holding means 44 may be different than what is shown in Fig. 9. A number of fluid bags 10 for future use may thus already be arranged at the apparatus 42. This makes it easier to attach a new fluid back 10 to the apparatus 42 via some conduit.

As shown in Fig. 9, the fluid bags 10 are arranged suspended from the holding means 44 such that the fluid bags are arranged after each other as seen in the thickness direction of the fluid bags 10. Thereby, the fluid bags 10 may be arranged in an efficient manner without consuming too much space.

As indicated above, Fig. 9 also illustrates a manner of using one or more fluid bags 10 according to the invention. One or more fluid bags 10 are according to this use thus arranged suspended from the holding means 44, such that the fluid bags 10 hang down from the holding means 44. The holding means 44 forms part of or is arranged in proximity to an apparatus 42 for hemodialysis, hemodiafiltration, hemofiltration or peritoneal dialysis.

The invention is not limited to the described embodiments but may be varied and modified within the scope of the following claims.

**Claims**

1. A fluid bag (10) comprising one or more compartments (12, 13) suitable for containing one or more fluids, the fluid bag (10) containing, in at least one compartment (12), or in a combination of different compartments (12, 13), either a dialysis fluid, suitable for use as a dialysis fluid in an apparatus (42) for hemodialysis, hemodiafiltration, hemofiltration or peritoneal dialysis, or a replacement fluid, suitable to be delivered to a patient in order to replace the ultrafiltrate that is withdrawn from a patient by a process of hemodialysis, hemofiltration, hemodiafiltration or peritoneal dialysis, or a fluid that is a rest product from a process of hemodialysis, hemofiltration, hemodiafiltration or peritoneal dialysis, the fluid bag (10) being at least partly made of a flexible material and comprises a first main sheet (14) and a second main sheet (16) located opposite to said first main sheet (14), the distance between said first (14) and second (16) sheets defining the thickness (t) of the fluid bag (10), the fluid bag (10) having a size such that it contains at least a certain quantity q ml of fluid when it is completely filled with one or more fluids, the fluid bag (10) is provided with one or more distance limiting members (40), arranged to limit the distance between said first (14) and second (16) sheets such that the thickness (t) of the fluid bag (10), when the fluid bag (10) is suspended such that it hangs vertically, never exceeds a certain value v mm independently of whether the fluid bag (10) is completely full, completely empty or filled to any degree there between, wherein:

$$q \geq 2000,$$

and

$$v \leq 2q/100.$$

2. A fluid bag (10) according to claim 1, wherein $v \leq$ 0.0175 q.

3. A fluid bag (10) according to claim 2, wherein $v \leq$ 0.016 q.

4. A fluid bag (10) according to any of the preceding claims, wherein $q \geq 3000$.

5. A fluid bag (10) according to claims 4, wherein $q \geq$ 4000.

6. A fluid bag (10) according to any of the preceding claims, wherein the extension of said first sheet (14) is limited by a first boundary (18, 20, 22, 24) of said first sheet (14), and wherein the extension of said second sheet is limited by a second boundary (18, 20, 22, 24) of said second sheet (16) and wherein at least one such distance limiting member (40) is formed by fastening said first sheet (14) to said second sheet (16) at a position located at least at a distance from said first and second boundaries (18, 20, 22, 24), and, if the bag includes a plurality of compartments (12, 13), at a distance from the borderlines (34) between the different compartments (12, 13).

7. A fluid bag (10) according to any of the preceding claims, wherein said at least one distance limiting member (40) is formed by a weld joining said first (14) and second (16) sheets.

8. A fluid bag (10) according to claim 7, wherein said weld (40) has the shape of a substantially straight line.

9. A fluid bag (10) according to claim 7, wherein said weld (40) has the shape of a loop.

10. A fluid bag (10) according to any of the preceding claims, wherein the fluid bag (10) includes a plurality of said distance limiting members (40).

11. A fluid bag (10) according to claim 10, wherein the fluid bag (10) includes at least three distance limiting members (40).

12. A fluid bag (10) according to any of the preceding claims, including at least two compartments (12, 13).

13. A fluid bag (10) according to claim 12 in combination with claim 10 or 11, wherein each of at least two compartments (12, 13) is provided with at least one distance limiting member (40).

14. A fluid bag (10) according to any of the preceding claims, wherein the fluid bag (10) has a first edge portion (18), and wherein the fluid bag (10) is provided with attachment means (30), located at said first edge portion (18), for attaching the fluid bag (10) to holding means (44), suitable to hold the fluid bag (10) in a suspended position.

15. A fluid bag (10) according to claim 14, wherein said attachment means (30) are formed by at least one hole (30) through said first edge portion (18).

16. A system for hemodialysis, hemodiafiltration, hemofiltration or peritoneal dialysis, the system comprising an apparatus (42) for hemodialysis, hemodiafiltration, hemofiltration or peritoneal dialysis and at least one fluid bag (10) according to any of the claims 1-15.

17. A system according to claim 16, comprising holding means (44) that forms part of or is arranged in proximity to said apparatus (42) for hemodialysis, hemodiafiltration, hemofiltration or peritoneal dialysis, wherein said fluid bag (10) is suspended by said holding means (44), such that the fluid bag (10) hangs down from said holding means (44).

18. A system according to claim 17, comprising a plurality of fluid bags (10), each fluid bag (10) being in accordance with any of the claims 1-15, wherein each fluid bag (10) is suspended by said holding means (44), such that each fluid bag (10) hangs down from said holding means (44).

19. A system according to claim 18, wherein said fluid bags (10) are arranged suspended from said holding means (44) such that the fluid bags (10) are arranged after each other as seen in the thickness direction of the fluid bags (10).

20. A system according to claim 19, wherein each fluid bag (10) is attached to said holding means (44) at a certain position at said holding means (44), and wherein the distance d mm between the positions for neighbouring fluid bags (10) is such that $d \geq v$.

**21.** A system according to claim 20, wherein v < d < 1.5 v.

**22.** A system according to claim 21, wherein v < d < 1.2 v.

**23.** A system according to any of the claims 30-33, wherein said holding means (44) includes a plurality of holding members (46) arranged to hold the fluid bags (10) suspended from said holding members (46).

**24.** A system according to claim 23, wherein each of said fluid bags (10) is a fluid bag (10) according to claim 15, and wherein for each fluid bag (10), said holding means (44) holds the fluid bag (10) by a holding member (46) protruding through said hole (30).

**25.** A system according to any of the claims 19-24, wherein the number of fluid bags (10) is at least three.

**26.** A system according to any of the claims 16-25, wherein at least one such fluid bag (10) is connected, via a conduit (48, 50, 52), to said apparatus (42) for hemodialysis, hemodiafiltration, hemofiltration or peritoneal dialysis.


**Patentansprüche**

**1.** Ein Fluidbeutel (10), welcher ein oder mehrere Fächer (12, 13) aufweist, welche geeignet sind, um ein oder mehrere Fluide zu enthalten, wobei der Fluidbeutel (10) in mindestens einem Fach (12) oder in einer Kombination von verschiedenen Fächern (12, 13) entweder eine Dialyseflüssigkeit, welche geeignet ist zur Verwendung als Dialyseflüssigkeit in einem Gerät (42) für Hämodialyse, Hämodiafiltration, Hämofiltration oder für peritoneale Dialyse, oder ein Substitutionsfluid, welches geeignet ist, zu einem Patienten geliefert zu werden, um das Ultrafiltrat zu ersetzen, welches von einem Patienten durch einen Prozess der Hämodialyse, Hämefiltration, Hämodiafiltration oder peritonealen Dialyse entzogen worden ist, oder ein Fluid, welches ein Restprodukt aus einem Prozess der Hämodialyse, Hämofiltration, Hämodiafiltration oder peritonealen Dialyse ist, enthält, wobei der Fluidbeutel (10) mindestens teilweise aus einem flexiblen Material besteht und einen ersten Hauptabschnitt (14) und einen zweiten Hauptabschnitt (16), welcher gegenüber dem ersten Hauptabschnitt (14) angeordnet ist, aufweist, wobei der Abstand zwischen dem ersten (14) und dem zweiten (16) Abschnitt die Dicke (t) des Fluidbeutels (10) bestimmt, wobei der Fluidbeutel (10) eine solche Größe hat, dass er mindestens eine bestimmte Menge q ml des Fluids enthält, wenn er vollständig mit einem oder mehreren Fluiden gefüllt ist, wobei der Fluidbeutel (10) mit einem oder mehreren abstandsbegrenzenden Bauteilen (40) versehen ist, welche derart angeordnet sind, um den Abstand zwischen dem ersten (14) und dem zweiten (16) Abschnitt zu begrenzen, derart, dass die Dicke (t) des Fluidbeutels (10), wenn der Fluidbeutel (10) derart aufgehängt ist, dass er vertikal hängt, niemals einen bestimmten Wert v mm überschreitet unabhängig davon, ob der Fluidbeutel (10) vollständig gefüllt ist, vollständig leer ist oder bis zu einem bestimmten Grad dazwischen gefüllt ist, wobei:

$$q \geq 2000,$$

und

$$v \leq 2q/100$$

ist.

**2.** Fluidbeutel (10) nach Anspruch 1, bei welchem v ≤ 0,0175 q ist.

**3.** Fluidbeutel (10) nach Anspruch 1, bei welchem v ≤ 0,0016 q ist.

**4.** Fluidbeutel (10) nach einem der vorhergehenden Ansprüche, bei welchem q ≥ 3000 ist.

**5.** Fluidbeutel (10) nach Anspruch 4, bei welchem q ≥ 4000 ist.

**6.** Fluidbeutel (10) nach einem der vorhergehenden Ansprüche, bei welchem die Ausdehnung des ersten Abschnittes (14) durch eine erste Grenze (18, 20, 22, 24) des ersten Abschnitts (14) begrenzt ist und bei welchem die Ausdehnung des zweiten Abschnitts durch eine zweite Grenze (18, 20, 22, 24) des zweiten Abschnitts (16) begrenzt ist und bei welchem mindestens ein solches abstandsbegrenzendes Bauteil (40) gebildet wird durch Befestigen des ersten Abschnitts (14) mit dem zweiten Abschnitt (16) an einer Position, welche mindestens in einem Abstand von der ersten und zweiten Grenze (18, 20, 22, 24) angeordnet ist, und, wenn der Beutel eine Vielzahl von Fächern (12, 13) aufweist, in einem Abstand von den Grenzlinien (34) zwischen den verschiedenen Fächern (12, 13).

**7.** Fluidbeuteil (10) nach einem der vorhergehenden Ansprüche, bei welchem das mindestens eine abstandsbegrenzende Bauteil (40) durch eine Schweißstelle gebildet ist, welche den ersten (14) und den zweiten (16) Abschnitt verbindet.

**8.** Fluidbeutel (10) nach Anspruch 7, bei welchem die Schweißstelle (40) die Form einer im Wesentlichen geraden Linie hat.

9. Fluidbeutel (10) nach Anspruch 7, bei welchem die Schweißstelle (40) die Form einer Schlaufe hat.

10. Fluidbeutel (10) nach einem der vorhergehenden Ansprüche, bei welchem der Fluidbeutel (10) eine Vielzahl von abstandsbegrenzenden Bauteilen (40) aufweist.

11. Fluidbeutel (10) nach Anspruch 10, bei welchem der Fluidbeutel (10) mindestens drei abstandsbegrenzende Bauteile (40) aufweist.

12. Fluidbeutel (10) nach einem der vorhergehenden Ansprüche, welcher mindestens zwei Fächer (12, 13) aufweist.

13. Fluidbeutel (10) nach Anspruch 12 in Kombination mit Anspruch 10 oder 11, bei welchem jedes der mindestens zwei Fächer (12, 13) mit mindestens einem abstandsbegrenzenden Bauteil (40) versehen ist.

14. Fluidbeutel (10) nach einem der vorhergehenden Ansprüche, bei welchem der Fluidbeutel (10) einen ersten Randabschnitt (18) hat und bei welchem der Fluidbeutel (10) mit Befestigungsmitteln (30) versehen ist, angeordnet an dem ersten Randabschnitt (18), zum Befestigen des Fluidbeutels (10) an einer Halteeinrichtung (44), geeignet, um den Fluidbeutel (10) in einer aufgehängten Position zu halten.

15. Fluidbeutel (10) nach Anspruch 14, bei welchem die Befestigungsmittel (30) durch mindestens ein Loch (30) durch den ersten Randabschnitt (18) gebildet werden.

16. Ein System für Hämodialyse, Hämodiafiltration, Hämofiltration oder peritoneale Dialyse, welches ein Gerät (42) für Hämodialyse, Hämodiafiltration, Hämofiltration oder peritoneale Dialyse und mindestens einen Fluidbeutel (10) nach einem der Ansprüche 1 bis 15 aufweist.

17. System nach Anspruch 16, welches eine Halteeinrichtung (44) aufweist, welche Teil oder in der Nähe des Gerätes (42) für Hämodialyse, Hämodiafiltration, Hämofiltration oder peritoneale Dialyse ist, wobei der Fluidbeutel (10) durch die Halteeinrichtung (44) aufgehängt wird, derart, dass der Fluidbeutel (10) von der Halteeinrichtung (44) nach unten hängt.

18. System nach Anspruch 17, welches eine Vielzahl von Fluidbeuteln (10) aufweist, wobei jeder Fluidbeutel (10) gemäß einem der Ansprüche 1 bis 15 ausgebildet ist, wobei jeder Fluidbeutel (10) durch die Halteeinrichtung (44) aufgehängt ist, derart, dass jeder Fluidbeutel (10) von der Halteeinrichtung (44) nach unten hängt.

19. System nach Anspruch 18, bei welchem die Fluidbeutel (10), aufgehängt an der Halteeinrichtung (44), derart angeordnet sind, dass die Fluidbeutel (10) hintereinander angeordnet sind, gesehen in der Dickenrichtung der Fluidbeutel (10).

20. System nach Anspruch 19, bei welchem jeder Fluidbeutel (10) an der Halteeinrichtung (44) an einer bestimmten Position an der Halteeinrichtung (44) befestigt ist und bei welchem der Abstand d mm zwischen den Positionen für benachbarte Fluidbeutel (10) derart ist, dass d ≥ v ist.

21. System nach Anspruch 20, bei welchem v < d < 1,5 v ist.

22. System nach Anspruch 21, bei welchem v < d < 1,2 v ist.

23. System nach einem der Ansprüche 20 bis 23, bei welchem die Halteeinrichtung (44) eine Vielzahl von Haltebauteilen (46) aufweist, angeordnet, um die Fluidbeutel (10), aufgehangen an den Haltebauteilen (46), zu halten.

24. System nach Anspruch 23, bei welchem jeder der Fluidbeutel (10) ein Fluidbeutel (10) gemäß Anspruch 15 ist und bei welchem für jeden Fluidbeutel (10) die Halteeinrichtung (44) den Fluidbeutel (10) durch ein Haltebauteil (46), welches das Loch (30) durchdringt, hält.

25. System nach einem der Ansprüche 19 bis 24, bei welchem die Anzahl der Fluidbeutel (10) mindestens drei ist.

26. System nach einem der Ansprüche 16 bis 25, bei welchem mindestens ein solcher Fluidbeutel (10) über eine Leitung (48, 50, 52) mit dem Gerät (42) für Hämodialyse, Hämediafiltration, Hämofiltration oder peritonealer Dialyse verbunden ist.

## Revendications

1. Poche à liquide (10) comportant un ou plusieurs compartiments (12, 13) aptes à contenir un ou plusieurs liquides, la poche à liquide (10) contenant, dans au moins un compartiment (12), ou en combinaison dans différents compartiments (12, 13), soit un fluide de dialyse, apte à être utilisé comme fluide de dialyse dans un appareil (42) pour l'hémodialyse, l'hémodiafiltration, l'hémofiltration ou la dialyse péritonéale, ou un liquide de remplacement, apte à être fourni à un patient de manière à remplacer l'ultrafiltrat qui est prélevé du patient par un procédé d'hémodialyse, d'hémofiltration, d'hémodiafiltration, ou de dialyse péritonéale, ou un liquide qui est un produit résidu d'un procédé d'hémodialyse, d'hémofiltration, d'hémodiafiltration, ou de dialyse péritonéale, la poche à liquide (10) étant au moins partiellement faite d'un matériau flexible et comportant une première feuille principale (14) et une deuxième feuille principale (16) disposée de manière opposée à ladite première feuille (14), la distance entre lesdites première (14) et deuxième (16) feuilles définissant l'épaisseur (t) de la poche à liquide (10), la poche à liquide (10) ayant une dimension telle qu'elle contient au moins une certaine quantité q ml lorsqu'elle est entièrement remplie avec un ou plusieurs liquides, la poche à liquide (10) étant munie d'un ou plusieurs éléments (40) réduisant la distance, disposés pour limiter la distance entre la première (14) et la deuxième (16) feuilles de telle manière que l'épaisseur (t) de la poche à liquide (10), lorsque la poche à liquide (10) est suspendue de manière qu'elle s'étende verticalement, ne dépasse jamais une certaine valeur v mm indépendamment du fait que la poche à liquide (10) est complètement remplie, complètement vide, ou remplie à un quelconque degré entre les deux, dans laquelle :

$$q \geq 2000$$

et

$$v \leq 2q/100$$

**2.** Poche à liquide (10) selon la revendication 1 dans laquelle $v \leq 0.0175\ q$.

**3.** Poche à liquide (10) selon la revendication 2 dans laquelle $v \leq 0.016\ q$.

**4.** Poche à liquide (10) selon l'une quelconque des revendications précédentes dans laquelle $q \geq 3000$.

**5.** Poche à liquide (10) selon la revendication 4 dans laquelle $q \geq 4000$.

**6.** Poche à liquide (10) selon l'une des revendications précédentes dans laquelle le prolongement de ladite première feuille (14) est limité par une première frontière (18, 20, 22, 24) de ladite première feuille (14) et dans laquelle le prolongement de ladite deuxième feuille est limité par une deuxième frontière (18, 20, 22, 24) de ladite deuxième feuille (16) et dans laquelle au moins un tel élément limitant la distance (40) est formé en attachant ladite première feuille (14) à ladite deuxième feuille (16) à une position située au moins à une distance depuis lesdites première et deuxième frontières (18, 20, 22, 24) et, si la poche comporte une pluralité de compartiments (12, 13), à une distance depuis les lignes de délimitation (34) entre les différents compartiments (12, 13).

**7.** Poche à liquide (10) selon l'une quelconque des revendications précédentes, dans laquelle ledit au moins un élément (40) limitant la distance est formé par une soudure reliant lesdites première (14) et deuxième (16) feuilles.

**8.** Poche à liquide (10) selon la revendication 7, dans laquelle ladite soudure (40) à la forme sensiblement d'une ligne droite.

**9.** Poche à liquide (10) selon la revendication 7, dans laquelle ladite soudure (40) a la forme d'une boucle.

**10.** Poche à liquide (10) selon l'une quelconque des revendications précédentes, dans laquelle ladite poche à liquide (10) comporte une pluralité dedits éléments (40) limitant la distance.

**11.** Poche à liquide (10) selon la revendication 10, dans laquelle la poche à liquide (10) comporte au moins trois éléments (40) limitant la distance.

**12.** Poche à liquide (10) selon l'une quelconque des revendications précédentes comportant au moins deux compartiments (12, 13).

**13.** Poche à liquide (10) selon la revendication 12 en combinaison avec la revendication 10 ou 11, dans laquelle chacun desdits au moins deux compartiments (12, 13) est muni d'au moins undit élément (40) limitant la distance.

**14.** Poche à liquide (10) selon l'une quelconque des revendications précédentes, dans laquelle la poche à liquide comporte une première portion de bord (18) et dans laquelle la poche à liquide (10) est munie de moyens de fixation (30) situés à ladite première portion de bord (18) pour attacher la poche à liquide 10 à des moyens de maintien (44) aptes à maintenir la poche à liquide (10) dans une position suspendue.

**15.** Poche à liquide (10) selon la revendication 14, dans laquelle lesdits moyens de fixation (30) sont constitués par au moins un trou (30) à travers ladite première portion de bord (18).

**16.** Système pour l'hémodialyse, hémodiafiltration, l'hémofiltration, ou la dialyse péritonéale, le système comportant un appareil (42) pour l'hémodialyse, l'hémodiafiltration, l'hémofiltration, ou la dialyse péritonéale et au moins une poche à liquide (10) selon l'une quelconque des revendications 1 à 15.

**17.** Système selon la revendication 16, comportant des moyens de maintien (44) qui forment une partie de, ou qui sont agencés à proximité dudit appareil (42) l'hémodialyse, l'hémodiafiltration, l'hémofiltration, ou la dialyse péritonéale, dans lequel ladite poche à liquide (10) est suspendue par lesdits moyens de maintien (44) de façon que la poche à liquide (10) soit suspendue vers le bas depuis lesdits moyens de maintien (44).

**18.** Système selon la revendication 17 comportant une pluralité de poches à liquide (10), chaque poche à liquide (10) étant conforme à l'une quelconque des revendications 1 à 15, dans lequel chaque poche à liquide (10) est suspendue par lesdits moyens de maintien (44) de façon que chaque poche à liquide (10) soit suspendue vers le bas à partir desdits moyens de maintien (44).

**19.** Système selon la revendication 18, dans lequel lesdites poches à liquide (10) sont agencées et suspendues depuis lesdits moyens de maintien (44) de façon que les poches à liquide soient agencées l'une après l'autre, vues selon la direction de l'épaisseur des poches à liquide (10).

**20.** Système selon la revendication 19, dans lequel chaque poche à liquide (10) est fixée auxdits moyens de maintien (44) dans une certaine position sur lesdits moyens de maintien (44), et dans lequel la distance d mm entre les positions de voisinage des poches à liquide (10) est telle que $d \geq v$.

**21.** Système selon la revendication 20, dans lequel v <1.5v.

**22.** Système selon la revendication 21, dans lequel v<d<1.2 v.

**23.** Système selon l'une quelconque des revendications 19 à 22, dans lequel lesdits moyens de maintien (44) comportent une pluralité d'éléments de maintien (46) agencés pour maintenir les poches à liquides (10) suspendues à partir desdits éléments de maintien (46).

**24.** Système selon la revendication 23, dans lequel chacune desdites poches à liquide (10) est une poche à liquide (10) selon la revendication 15, et dans lequel, pour chaque poche à liquide (10), lesdits moyens de maintien (44) maintiennent la poche à liquide (10) au moyen d'un élément de maintien faisant saillie à travers ledit trou (30).

**25.** Système selon l'une quelconque des revendications 19 à 24, dans lequel le nombre de poches à liquide (10) est égal à au moins trois.

**26.** Système selon l'une quelconque des revendications 16 à 25 dans lequel au moins une dite poche à liquide 10 est connectée, via un conduit (40, 50, 52) audit appareil (42) pour l'hémodialyse, l'hémodiafiltration, l'hémofiltration ou la dialyse péritonéale.

Fig 1

Fig 2

Fig 3

Fig 4

Fig 5

Fig 6

Fig 7

14

Fig 8

Fig 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 904789 A2
- WO 9927885 A1
- US 5364385 A
- EP 711536 A1